# EUROPEAN PATENT SPECIFICATION

(11) **EP 4 566 577 B1**
(45) Date of publication and mention of the grant of the patent: **29.04.2026**
(21) Application number: 24213363.5
(22) Date of filing: 15.11.2024
(51) Int. Cl.: A61F 13/15

(54) **AN APPARATUS FOR TREATING CONTINUOUS MOVING WEBS AND A MACHINE COMPRISING THIS APPARATUS**
VORRICHTUNG ZUR BEHANDLUNG VON SICH KONTINUIERLICH BEWEGENDEN BAHNEN UND MASCHINE MIT DIESER VORRICHTUNG
APPAREIL POUR TRAITER DES BANDES CONTINUES EN MOUVEMENT ET MACHINE COMPRENANT CET APPAREIL

(30) Priority: 05.12.2023 IT 202300025977
(43) Date of publication of application: 11.06.2025
(73) Proprietor: Fameccanica.Data S.p.A., 66020 San Giovanni Teatino (CH) (IT)
(72) Inventor: COCOZZELLA, Nicola, 66020 San Giovanni Teatino (Chieti) (IT); SEPE, Davide, 66020 San Giovanni Teatino (Chieti) (IT)
(74) Representative: Buzzi, Notaro & Antonielli d'Oulx S.p.A.

(56) References cited:
- US-A1- 2012 247 661
- US-A1- 2023 150 159
- US-A1- 2023 234 254

## Description

### Field of the invention

The present invention relates in general to treating continuous moving webs.

More specifically, the invention relates to an apparatus that can be used to perform operations of cutting, welding, embossing, drilling, etc. on continuous moving webs, for example, on composite webs comprising one or more non-woven layers.

The invention was developed in particular for its application to producing absorbent sanitary articles, such as diapers, diaper-pants, absorbent articles for incontinent adults, etc., but is not limited to this specific sector.

According to another aspect, the invention concerns a machine comprising an apparatus for treating continuous moving webs.

### Description of the prior art

For producing absorbent sanitary articles, it is frequently necessary to carry out various types of processing on continuous moving webs, for example, continuous composite webs comprising a plurality of elastic threads enclosed between one or more non-woven layers. Processing operations may comprise, for example, cutting, welding, embossing, drilling, etc.

These operations are normally carried out using an apparatus comprising an anvil roller and a tool holder roller on which tools such as cutting blades, welding elements, embossing elements, etc. are arranged depending on the processing operations to be carried out.

Rotary machines for treating continuous moving webs are configured to operate on webs corresponding to a specific format of absorbent sanitary articles.

When the format of the absorbent sanitary articles produced on the same line changes, the width of the operations that must be carried out on the continuous webs used for producing absorbent sanitary articles often also changes.

Normally, when the format of the absorbent sanitary articles changes, the rotary tools that perform the operations of cutting, welding, embossing, etc. must be replaced to adapt the width of the tools to the new size of the articles to be produced.

The solutions currently adopted to carry out a format change involve the complete or partial replacement of the rotary unit.

Changing the format is one of the most complex and time-consuming activities, as it requires disengagement of the rotary axes transmissions, pneumatic connections and mechanical fastenings of the unit, in particularly difficult-to-reach points.

Furthermore, extraction of the unit requires that there is access space in the line for a lifting device such as a forklift truck, which severely limits the possibilities for using the space in front of the machine.

In some cases, it may be necessary to replace only the tool holder roller of the apparatus, but this also involves the need to carry out very delicate adjustment operations (in the order of microns), which may lead to the risk of damaging the tools. Documents US 2023/234254 A1, US 2012/247661 A1 and US 2023/150159 A1 disclose apparatuses for carrying operations on a continuous sheet used for the production of absorbent sanitary articles, wherein there is no need for a complete or partial replacement of the rotary unit when a format change takes place.

### Object and summary of the invention

The object of the present invention is to provide an apparatus for treating continuous moving webs that overcomes the problems of the prior art

According to the present invention, this object is achieved by an apparatus having the characteristics of claim 1.

According to another aspect, the invention relates to a machine according to claim 10.

Preferred embodiments of the invention form the subject of the dependent claims.

The claims form an integral part of the disclosure provided here in relation to the invention.

### Brief description of the drawings

The present invention will now be described in detail with reference to the attached drawings, given purely by way of non-limiting example, wherein:
- Figure 1 is a schematic front view of a first embodiment of an apparatus according to the present invention,
- Figure 2 is a schematic side view according to the arrow II of Figure 1,
- Figure 3 is a schematic front view of a second embodiment of an apparatus according to the present invention,
- Figure 4 is a schematic side view according to the arrow IV of Figure 3, and
- Figures 5 and 6 are schematic plan views of two continuous webs processed by an apparatus according to the present invention in a first operating condition and in a second operating condition, respectively.

### Detailed description

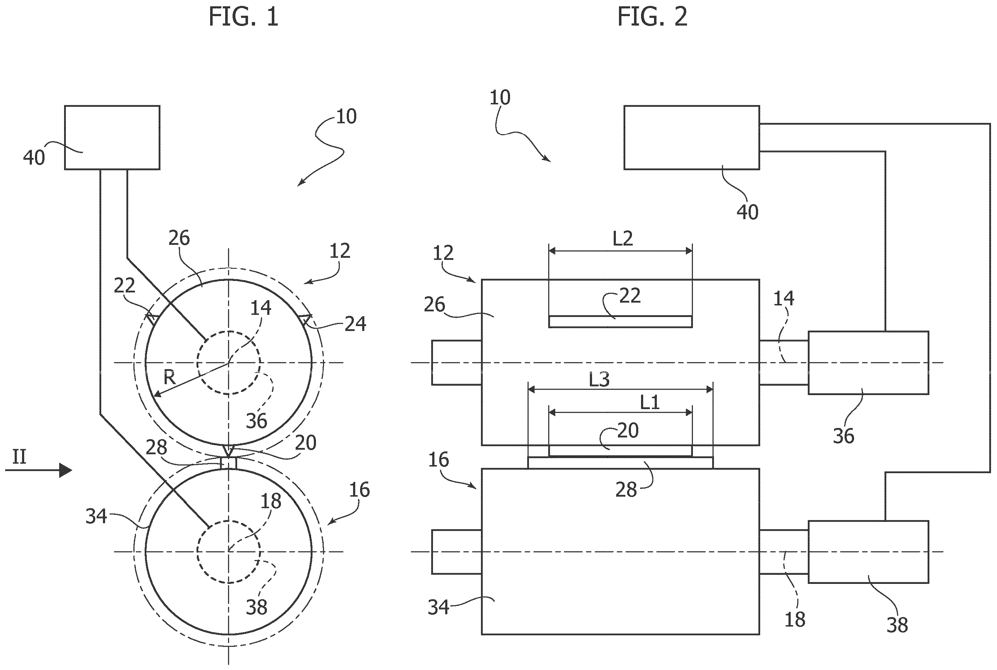

With reference to Figures 1-4, numeral 10 indicates an apparatus for treating a continuous moving web.

The apparatus 10 comprises a tool holder roller 12 rotatable around a first rotation axis 14 and an anvil roller 16 rotatable around a second rotation axis 18 parallel to the first rotation axis 14.

The tool holder roller 12 carries at least one first tool 20 and at least one second tool 22 spaced angularly apart from each other. In general, the tool holder roller 12 may carry a generic number of tools equal to or greater than 2. In the example illustrated in the Figures, the tool holder roller 12 carries a first tool 20, a second tool 22 and a third tool 24.

Below reference will be made, by way of example, to the case wherein the tool holder roller 12 carries a first tool 20 and a second tool 22, but it is understood that what will be described applies equally to the case wherein the tool holder roller 12 carries a generic number of tools equal to or greater than 2.

The first tool 20 and the second tool 22 may be configured to perform an operation chosen from: cutting, welding, embossing, laminating, and perforating.

The first tool 20 and the second tool 22 have different geometric characteristics. In particular, the first tool 20 and the second tool 22 may have respective different lengths L1, L2.

The first tool 20 and the second tool 22 may have active surfaces configured to perform different processing operations, for example, the same type of machining but with different patterns or different processing operations, e.g. welding, embossing, etc.

In possible embodiments, the first tool 20 and the second tool 22 protrude radially from an outer surface 26 of the tool holder roller.

The anvil roller 16 carries at least one anvil element 28.

In the embodiment illustrated in Figures 1 and 2 the anvil roller **16** carries a single anvil element 28. In this case, as will be described below, the first tool 20, the second tool 22 and any additional tools 24 are intended to cooperate with the same anvil element 28. The single anvil element 28 has a width L3 greater than the maximum width of the tools 20, 22, 24.

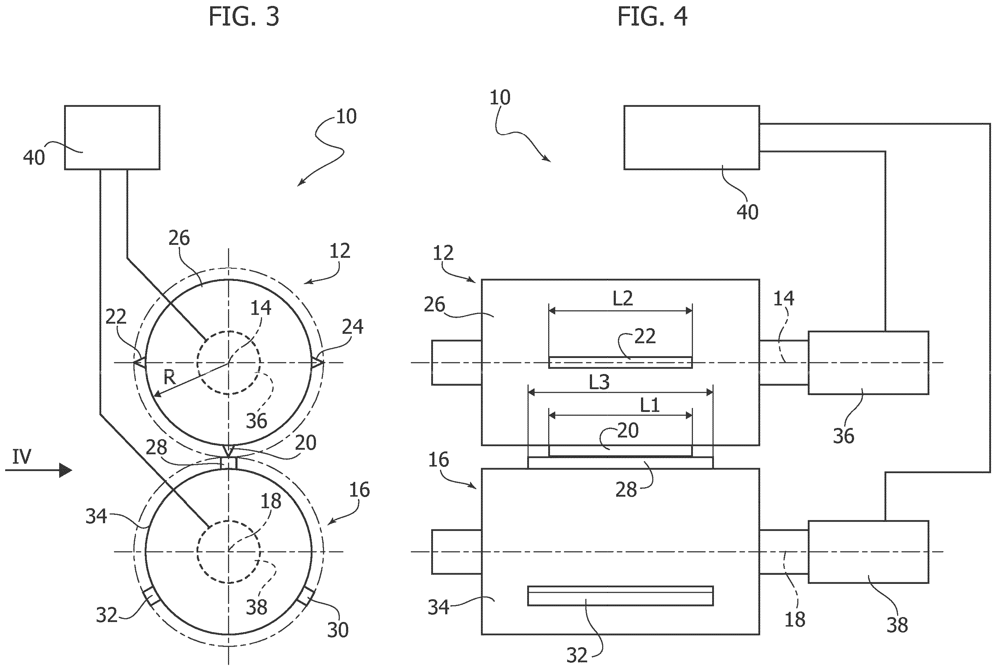

In the embodiment illustrated in Figures 3 and 4 the anvil roller 16 carries a plurality of anvil elements 28, 30, 32 spaced angularly apart from each other. In this case, as will be described below, the first tool 20, the second tool 22 and any additional tools 24 are intended to cooperate with respective anvil elements 28, 30, 32. In this case each tool 20, 22, 24 may be set with the corresponding anvil element 28, 30, 32.

Each of the anvil elements 28, 30, 32 may protrude radially from an outer surface 34 of the anvil roller **16.**

The tool holder roller 12 and the anvil roller **16** are driven by respective electric motors 36, 38 controlled by a control device 40.

The control device 40 is configured to rotate the tool holder roller 12 and the anvil roller **16** about their respective rotation axes 14, 18 in at least one first and one second operating condition. The number of operating conditions may be equal to the number of different tools 20, 22, 24 provided on the tool holder roller **12.**

In the first operating condition, the first tool 20 of the tool holder roller 12 is in phase with the single anvil element 28 or with a respective anvil element 28, 30, 32 and, during operation, it cooperates cyclically with the single anvil element 28 or with a respective anvil element 28, 30, 32 to carry out a processing operation on a continuously moving web. In the first operating condition, the second tool 22 and any additional tool 24 are out of phase with respect to the single anvil element 28 or with respect to the plurality of anvil elements 28, 30, 32 and, during operation, do not cooperate with any anvil element 28, 30, 32.

In the second operating condition, the second tool 22 of the tool holder roller 12 is in phase with the single anvil element 28 or with a respective anvil element 28, 30, 32 and, during operation, it cooperates cyclically with the single anvil element 28 or with a respective anvil element 28, 30, 32 to carry out a processing operation on a continuously moving web. In the second operating condition, the first tool 20 and any additional tool 24 are out of phase with respect to the single anvil element 28 or with respect to the plurality of anvil elements 28, 30, 32 and, during operation, do not cooperate with any anvil element 28, 30, 32.

Therefore, the apparatus 10 may operate in different operating conditions in each of which only one type of tool is operational and may switch from one operating condition to another with a software selection.

The transition from one operating condition to another gives the possibility of changing the geometry of the processing carried out on a continuous moving web and corresponds to a change in format of the apparatus **10.**

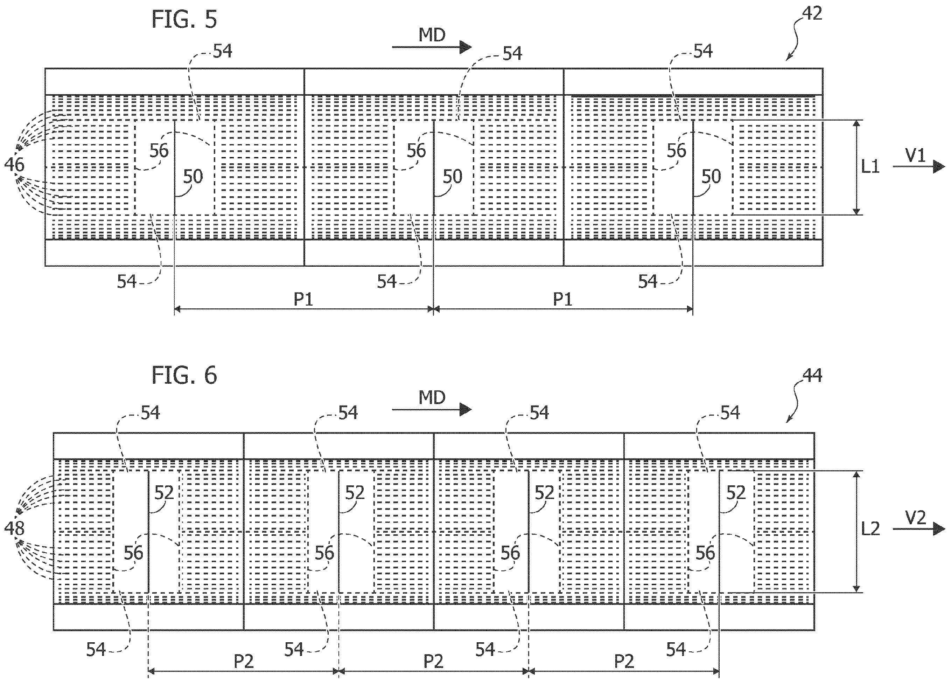

Figures 5 and 6 show two continuous webs 42, 44 processed by the apparatus 10 in the first and second operating conditions, respectively. In the example illustrated, the continuous webs 42, 44 are elastic bands comprising a plurality of longitudinal elastic threads 46, 48 enclosed between two non-woven layers. The longitudinal elastic threads 46, 48 are attached between the two continuous webs 42, 44 by means of discontinuously applied adhesive. Lines 54 and 56 define windows in which no adhesive is applied and in which the longitudinal elastic threads 46, 48 are not attached between the two continuous webs 42, 44.

In the illustrated example, the continuous webs 42, 44 are subjected to cutting operations in the apparatus 10 to cut the longitudinal elastic threads 46, 48 transversely at regular intervals. In Figures 5 and 6 the transverse cuts are indicated with 50 and 52 and have respective widths L1 and L2. After the transverse cuts 50, 52, the longitudinal elastic threads 46, 48 in the sections wherein they are not attached to the two continuous webs 42, 44 retract to the respective lines 56.

In the first operating condition, the apparatus 10 is configured to operate with a first tool 20 having a width L1, which cooperates cyclically with one of the anvil elements 28, 30, 32 to carry out first cuts 50 on the first moving continuous web 42 with width L1 and with a pitch P1.

In the second operating condition, the apparatus 10 is configured to operate with a second tool 22 having a width L2, which cooperates cyclically with one of the anvil elements 28, 30, 32 to carry out second cuts 52 on the second moving continuous web 44 with width L2 and with a pitch P2.

The change of operating conditions is done exclusively via software, without the need to replace components and without the need to make settings or adjustments.

The control device 40 controls the tool holder roller 12 and the anvil roller 16 in phase with each other so as to carry out the operations, in this case the cuts 50 or 52, with the required pitches P1, P2.

The rotation speeds of the tool holder roller 12 and the anvil roller **16** may vary during each single rotation to carry out operations with programmed pitches P1, P2.

When the tool 20, 22, 24, which is operational at any given time, cooperates with a respective anvil element 28, 30, 32, the speeds of the tool 20, 22, 24 and of the anvil element 28, 30, 32 may be equal to the feed rate V1, V2 of the continuous web 42, 44 in the machine direction MD. After having performed an operation, the tool 20, 22, 24 operating at any given time and the respective anvil element 28, 30, 32 may accelerate or decelerate to perform the next operation after the continuous web 42, 44 has advanced in the machine direction MD by a pre-established pitch P1, P2. The tools that are not operational contact the continuous web 42, 44 out of phase with respect to the anvil elements 28, 30, 32 and do not perform any operation on the web 42, 44.

The apparatus 10 may be configured to carry out intermittent operations on the moving continuous webs 42, 44 having respective extensions in the longitudinal direction of the continuous webs 42, 44.

Each operation has an extension in the direction of movement of the web (machine direction MD) that is less than the size of the circumference of the anvil roller 12 divided by the number of possible format changes. For example, if the anvil roller 12 has a radius R and carries n tools, each operation may have a dimension in the machine direction MD less than 2nR/n.

It will be understood that it is possible to combine multiple apparatuses 10 to perform different operations or the same operation with different widths.

The invention also relates to a machine for producing absorbent sanitary articles, such as diapers, diaper-pants, absorbent articles for incontinent adults, etc. comprising an apparatus for treating continuous moving webs as previously described.

Of course, without prejudice to the principle of the invention, the details of construction and the embodiments can be widely varied with respect to those described and illustrated, without thereby departing from the scope of the invention as defined by the claims that follow.

## Claims

1. An apparatus for treating a continuous moving web (42, 44), comprising:
- a tool holder roller (12) rotatable around a first rotation axis (14) and carrying at least one first tool (20) and at least one second tool (22) spaced angularly apart from each other and having geometric characteristics different from each other,
- an anvil roller (16) rotatable around a second rotation axis (18) parallel to the first rotation axis (14) and carrying at least one anvil element (28, 30, 32),
- a control device (40) configured to rotate the tool holder roller (12) and the anvil roller (16) around the respective rotation axes (14, 18) in at least one first operating condition and one second operating condition, wherein in the first operating condition said first tool (20) is in phase and cooperates cyclically with at least one anvil element (28, 30, 32) and said at least one second tool (22) is out of phase and does not cooperate with an anvil element (28, 30, 32), and wherein in the second operating condition said second tool (22) is in phase and cooperates cyclically with at least one anvil element (28, 30, 32) and said first tool (20) is out of phase and does not cooperate with an anvil element (28, 30, 32) .

2. An apparatus according to claim **1,** wherein said first tool (20) and second tool (22) have respective lengths (L1, L2) different from each other.

3. An apparatus according to claim 1 or claim 2, wherein said first tool (20) and second tool (22) are configured to perform operations selected from: cutting, welding, laminating, embossing, and perforating.

4. An apparatus according to any of the preceding claims, wherein said first tool (20) and second tool (22) have respective active surfaces configured to perform operations different from each other.

5. An apparatus according to any of the preceding claims, wherein said first tool (20) and second tool (22) in said first and second operating conditions cooperate with the same anvil element (28).

6. An apparatus according to any of claims 1-4, wherein the anvil roller (16) carries a plurality of anvil elements (28, 30, 32) spaced angularly apart from each other and wherein in said first and second operating conditions said first tool (20) and second tool (22) cooperate with respective anvil elements (28, 30, 32) .

7. An apparatus according to any of the preceding claims, wherein said first tool (20) and second tool (22) protrude in a radial direction from an outer surface (26) of the tool holder roller (12).

8. An apparatus according to any of the preceding claims, wherein said at least one anvil element (28, 30, 32) protrudes in a radial direction from an outer surface (34) of the anvil roller (16).

9. An apparatus according to any of the preceding claims, wherein said control device (40) is configured to vary the rotation speeds of the tool holder roller (12) and the anvil roller (16) during each single rotation to carry out operations with programmed pitches (P1, P2).

10. A machine comprising an apparatus for treating a continuous moving web (42, 44) as claimed in any of claims 1-9.

## Patentansprüche

1. Eine Vorrichtung zur Behandlung einer kontinuierlich bewegten Bahn (42, 44), umfassend:
- einen Werkzeughalterzylinder (12), der um eine erste Drehachse (14) drehbar ist und mindestens ein erstes Werkzeug (20) und mindestens ein zweites Werkzeug (22) trägt, die in Umfangsrichtung voneinander beabstandet sind und unterschiedliche geometrische Eigenschaften aufweisen,
- einen Ambosszylinder (16), der um eine zweite Drehachse (18) parallel zur ersten Drehachse (14) drehbar ist und mindestens ein Ambosselement (28, 30, 32) trägt,
- eine Steuereinrichtung (40), die konfiguriert ist, um den Werkzeughalterzylinder (12) und den Ambosszylinder (16) um die jeweiligen Drehachsen (14, 18) in mindestens einem ersten Betriebszustand und einem zweiten Betriebszustand zu drehen, wobei im ersten Betriebszustand besagtes erstes Werkzeug (20) in Phase ist und zyklisch mit mindestens einem Ambosselement (28, 30, 32) zusammenwirkt und besagtes mindestens eine zweites Werkzeug (22) außer Phase ist und nicht mit einem Ambosselement (28, 30, 32) zusammenwirkt, und wobei im zweiten Betriebszustand besagtes zweites Werkzeug (22) in Phase ist und zyklisch mit mindestens einem Ambosselement (28, 30, 32) zusammenwirkt und besagtes erstes Werkzeug (20) außer Phase ist und nicht mit einem Ambosselement (28, 30, 32) zusammenwirkt.

2. Eine Vorrichtung nach Anspruch 1, wobei besagtes erstes Werkzeug (20) und zweites Werkzeug (22) jeweilige Längen (L1, L2) aufweisen, die voneinander verschieden sind.

3. Eine Vorrichtung nach Anspruch 1 oder Anspruch 2, wobei besagtes erstes Werkzeug (20) und zweites Werkzeug (22) konfiguriert sind, um Operationen auszuwählen aus: Schneiden, Schweißen, Laminieren, Prägen und Perforieren.

4. Eine Vorrichtung nach einem der vorhergehenden Ansprüche, wobei besagtes erstes Werkzeug (20) und zweites Werkzeug (22) jeweilige aktive Oberflächen aufweisen, die konfiguriert sind, um voneinander verschiedene Operationen durchzuführen.

5. Eine Vorrichtung nach einem der vorhergehenden Ansprüche, wobei besagtes erstes Werkzeug (20) und zweites Werkzeug (22) in besagten ersten und zweiten Betriebszuständen mit demselben Ambosselement (28) zusammenwirken.

6. Eine Vorrichtung nach einem der Ansprüche 1-4, wobei der Ambosszylinder (16) eine Vielzahl von Ambosselementen (28, 30, 32) trägt, die in Umfangsrichtung voneinander beabstandet sind, und wobei in besagten ersten und zweiten Betriebszuständen besagtes erstes Werkzeug (20) und zweites Werkzeug (22) mit jeweiligen Ambosselementen (28, 30, 32) zusammenwirken.

7. Eine Vorrichtung nach einem der vorhergehenden Ansprüche, wobei besagtes erstes Werkzeug (20) und zweites Werkzeug (22) in einer radialen Richtung von einer Außenfläche (26) des Werkzeughalterzylinders (12) vorstehen.

8. Eine Vorrichtung nach einem der vorhergehenden Ansprüche, wobei besagtes mindestens eine Ambosselement (28, 30, 32) in einer radialen Richtung von einer Außenfläche (34) des Ambosszylinders (16) vorsteht.

9. Eine Vorrichtung nach einem der vorhergehenden Ansprüche, wobei besagte Steuereinrichtung (40) konfiguriert ist, um die Drehgeschwindigkeiten des Werkzeughalterzylinders (12) und des Ambosszylinders (16) während jeder einzelnen Umdrehung zu variieren, um Operationen mit programmierten Teilungen (P1, P2) durchzuführen.

10. Eine Maschine, umfassend eine Vorrichtung zur Behandlung einer kontinuierlich bewegten Bahn (42, 44) nach einem der Ansprüche 1-9.

## Revendications

1. Un dispositif pour traiter une bande continue en mouvement (42, 44), comprenant :
- un rouleau porte-outil (12) pouvant tourner autour d'un premier axe de rotation (14) et portant au moins un premier outil (20) et au moins un deuxième outil (22) espacés angulairement l'un de l'autre et ayant des caractéristiques géométriques différentes l'une de l'autre,
- un rouleau enclume (16) pouvant tourner autour d'un deuxième axe de rotation (18) parallèle au premier axe de rotation (14) et portant au moins un élément enclume (28, 30, 32),
- un dispositif de commande (40) configuré pour faire tourner le rouleau porte-outil (12) et le rouleau enclume (16) autour des axes de rotation respectifs (14, 18) dans au moins une première condition de fonctionnement et une deuxième condition de fonctionnement, dans lequel, dans la première condition de fonctionnement, ledit premier outil (20) est en phase et coopère cycliquement avec au moins un élément enclume (28, 30, 32) et ledit au moins deuxième outil (22) est hors phase et ne coopère pas avec un élément enclume (28, 30, 32), et dans lequel, dans la deuxième condition de fonctionnement, ledit deuxième outil (22) est en phase et coopère cycliquement avec au moins un élément enclume (28, 30, 32) et ledit premier outil (20) est hors phase et ne coopère pas avec un élément enclume (28, 30, 32).

2. Un appareil selon la revendication 1, dans lequel ledit premier outil (20) et ledit deuxième outil (22) ont des longueurs respectives (L1, L2) différentes l'une de l'autre.

3. Un appareil selon la revendication 1 ou la revendication 2, dans lequel ledit premier outil (20) et ledit deuxième outil (22) sont configurés pour effectuer des opérations sélectionnées parmi : couper, souder, laminer, gaufrer et perforer.

4. Un appareil selon l'une quelconque des revendications précédentes, dans lequel ledit premier outil (20) et ledit deuxième outil (22) ont des surfaces actives respectives configurées pour effectuer des opérations différentes l'une de l'autre.

5. Un appareil selon l'une quelconque des revendications précédentes, dans lequel ledit premier outil (20) et ledit deuxième outil (22) dans lesdites première et deuxième conditions de fonctionnement coopèrent avec le même élément enclume (28).

6. Un appareil selon l'une quelconque des revendications 1 à 4, dans lequel le rouleau enclume (16) porte une pluralité d'éléments enclume (28, 30, 32) espacés angulairement les uns des autres et dans lequel, dans lesdites première et deuxième conditions de fonctionnement, ledit premier outil (20) et ledit deuxième outil (22) coopèrent avec des éléments enclume respectifs (28, 30, 32).

7. Un appareil selon l'une quelconque des revendications précédentes, dans lequel ledit premier outil (20) et ledit deuxième outil (22) font saillie dans une direction radiale depuis une surface extérieure (26) du rouleau porte-outil (12).

8. Un appareil selon l'une quelconque des revendications précédentes, dans lequel ledit au moins un élément enclume (28, 30, 32) fait saillie dans une direction radiale depuis une surface extérieure (34) du rouleau enclume (16).

9. Un appareil selon l'une quelconque des revendications précédentes, dans lequel ledit dispositif de commande (40) est configuré pour faire varier les vitesses de rotation du rouleau porte-outil (12) et du rouleau enclume (16) pendant chaque rotation unique pour effectuer des opérations avec des pas programmés (P1, P2).

10. Une machine comprenant un appareil pour traiter une bande continue en mouvement (42, 44) selon l'une quelconque des revendications 1 à 9.
